# FASCICULE DE BREVET EUROPEEN

(11) **EP 0 433 429 B1**
(45) Date de publication et mention de la délivrance du brevet: **25.01.1995**
(21) Numéro de dépôt: 90910755.9
(22) Date de dépôt: 12.07.1990
(51) Int. Cl.: A61M 31/00

(54) **DISPOSITIF DE DELIVRANCE D'UN PRINCIPE ACTIF PHARMACOLOGIQUE PAR POMPAGE ELECTROLYTIQUE**
VORRICHTUNG ZUR LIEFERUNG EINES AKTIVEN PHARMAZEUTISCHEN PRINZIPS DURCH ELEKTROLYTISCHES PUMPEN
DEVICE FOR RELEASING A PHARMACOLOGICALLY ACTIVE PRINCIPLE BY ELECTROLYTIC PUMPING

(30) Priorité: 12.07.1989 FR 8909391
(43) Date de publication de la demande: 26.06.1991
(73) Titulaire: APCIS, F-51100 Reims (FR)
(72) Inventeur: TAFANI, Jean-Pierre, F-75019 Paris (FR); VALTER, Francis, F-92190 Chatenay-Malabry (FR); ZEGHAL, Slim, F-75012 Paris (FR); ALEXANDRE, Jean, F-75020 Paris (FR)
(74) Mandataire: Warcoin, Jacques
(86) Numéro de dépôt international: FR9000526
(87) Numéro de publication internationale: WO9100753

(56) Documents cités:
- FR-A- 2 195 461
- FR-A- 2 305 197
- US-A- 3 115 280

## Description

La présente invention concerne un dispositif de délivrance d'un principe actif pharmacologique par pompage électrolytique.

Un tel dispositif est par exemple décrit dans le FR-A-2 195 461 qui propose, dans l'un de ses modes de réalisation, une pompe à effet électrolytique comportant une première chambre à volume variable, close, renfermant un électrolyte solide (électrolyte gélifié) produisant un dégagement gazeux sous l'effet d'une tension appliquée à une paire d'électrodes; ce dégagement gazeux provoque une expansion de la première chambre, qui a une forme générale de soufflet.

Cette pompe comporte également une seconde chambre à volume variable, renfermant le principe actif et débouchant vers l'extérieur par un orifice permettant la délivrance de celui-ci; une enceinte rigide définit le volume total des deux chambres, qui est constant, de sorte que le gonflement du soufflet provoque une diminution de volume de la seconde chambre et une expulsion corrélative, contrôlée, du principe actif que renferme cette dernière.

L'une des principales difficultés rencontrée avec les pompes à effet électrolytique de ce type est le contrôle du débit de principe actif.

En effet, le phénomène d'électrolyse est un processus par nature évolutif, compte tenu de la modification graduelle des propriétés physico-chimiques de l'électrolyte (résistance propre, notamment) et des électrodes (polarisation).

La structure de la pompe précitée ne permet pas d'obtenir un débit de principe actif qui soit à la fois exact, précis et stable.

En outre, la structure de pompe proposée par ce document est essentiellement prévue pour une délivrance en continu du principe actif, pendant des périodes relativement longues. En effet, du fait de l'utilisation d'un électrolyte solide, la constante de temps de la cellule d'électrolyse est relativement élevée en raison de la nécessaire diffusion au travers du gel. Cette structure de pompe électrolytique ne permet donc pas l'expulsion de doses ponctuelles, très brèves, ni a fortiori de doses brèves et précises.

En effet, de nouveaux principes actifs tels que l'hormone de croissance, son releasing factor, de nombreux peptides, hormones, médiateurs chimiques, certains antibiotiques et certains antimitotiques nécessitent, pour préserver leur efficacité thérapeutique ou pour diminuer les effets secondaires, des injections discontinues et fréquentes et non une perfusion continue de produit à débit fixé et constant dans le temps.

La caractéristique de débit du principe actif injecté en fonction du temps doit pour cela présenter des pics très étroits, qui sont impossibles à réaliser si le dispositif présente une constante de temps excessive, comme cela serait le cas avec un électrolyte solide.

En revanche, l'emploi d'un électrolyte solide résoud le problème de la permanence du contact entre électrodes et électrolyte puisque le gaz dégagé par le phénomène d'électrolyse diffuse hors du gel et ne vient pas s'interposer entre les électrodes et l'électrolyte, ce qui pourrait être le cas avec un électrolyte liquide (notamment dans le cas d'un dispositif implanté ou ingéré qui, à un moment donné, peut avoir n'importe quelle orientation dans l'espace).

L'état de la technique peut être complété par le document US-3 115 280 qui enseigne un dispositif du type comprenant une cellule d'électrolyse comportant une première chambre à volume variable, close, affectant une forme tubulaire renfermant un électrolyte liquide baignant des electrodes de forme allongée et produisant un dégagement gazeux sous l'effet d'une tension d'électrolyse appliquée au niveau des électrodes en contact avec cet électrolyte, ce dégagement gazeux provoquant une expansion de la première chambre, et une seconde chambre à volume variable renfermant le principe actif, cette chambre débouchant vers l'extérieur par un orifice permettant la délivrance du principe actif, le volume total des deux chambres étant constant, de sorte que l'expansion de la première chambre provoque une diminution de volume de la seconde chambre et une expulsion corrélative, contrôlée, du principe actif que renferme cette dernière, les deux chambres étant séparées l'une de l'autre par une paroi souple subissant la pression résultant du dégagement gazeux de manière à réduire le volume de la seconde chambre et en expulser le principe actif.

L'inconvénient d'un tel dispositif est que dans certaines positions, les électrodes ne se trouvent plus en contact avec l'électrolyte.

La présente invention se propose de remédier à ces inconvénients en proposant une pompe à effet électrolytique dont la structure permette un contrôle extrêmement précis du débit de principe actif, avec en outre une très grande constance au cours du temps.

Un autre but de la présente invention est de proposer une structure adaptée à l'éjection séquentielle de doses ponctuelles, c'est-à-dire précises et brèves (typiquement, pendant une durée de quelques secondes ou quelques fractions de seconde).

L'invention a également pour objet la réalisation d'un dispositif qui, du fait de sa configuration propre, soit aisément implantable ou ingestible, afin de permettre la délivrance in situ du principe actif.

A cet effet, l'invention propose un dispositif de délivrance d'un principe actif pharmacologique par pompage électrolytique du même type que celui de l'US-3 115 280 précités.

Selon l'invention, ce dispositif est caractérisé en ce que les électrodes de forme allongée sont disposées contre la paroi intérieure du tube et s'étendent sur la majeure partie de la longueur de cette première chambre de manière à se trouver toujours au moins partiellement en contact avec l'électrolyte quelle que soit la position du dispositif, et que la seconde chambre à volume variable s'étend axialement sur la majeure partie de la longueur de ladite première chambre.

Dans une forme de réalisation préférentielle, la première chambre est définie par un tube rigide fermé à l'une de ses extrémités, ce tube enfermant une poche souple centrale s'étendant axialement sur la majeure partie de la longueur du tube et définissant la seconde chambre, le rebord de cette poche étant relié au tube rigide à l'extrémité opposée à son extrémité fermée et étant fermé par un obturateur comportant l'orifice de délivrance du principe actif.

Dans ce cas, les électrodes sont de préférence des électrodes de forme allongée disposée contre la paroi intérieure du tube et s'étendant sur la majeure partie de la longueur de celui-ci. De préférence, il est alors prévu une pluralité de paires d'électrodes semblables, réparties circonférentiellement de façon régulière contre la paroi intérieure du tube avec alternance des polarités d'une électrode à la suivante.

De préférence, il est prévu, en série dans le circuit de la cellule d'électrolyse, une résistance de valeur notablement supérieure à la résistance propre, prise entre les électrodes, de l'électrolyte.

L'invention propose en outre d'appliquer la tension d'électrolyse aux électrodes de façon intermittente, de manière à délivrer le principe actif par doses prédéterminées répétées, administrées à intervalles réguliers.

A cet effet, le dispositif de l'invention comporte alors un circuit de contrôle d'électrolyse comprenant: une source de tension d'électrolyse; un oscillateur, délivrant une fréquence de référence prédéterminée; des premiers moyens de comptage, recevant en entrée la fréquence de référence et délivrant en sortie un signal périodique de séquencement définissant la périodicité d'administration de la dose de principe actif; des seconds moyens de comptage, recevant en entrée la fréquence de référence et délivrant en sortie un signal périodique d'activation définissant la durée d'administration de chaque dose de principe actif; et un circuit de commande, combinant ces signaux de séquencement et d'activation de manière à commander sélectivement l'application de la tension d'électrolyse aux bornes de la cellule d'électrolyse de manière à réaliser la délivrance du principe actif avec la périodicité et la durée voulues.

Ce circuit de contrôle d'électrolyse peut en outre comprendre des troisièmes moyens de comptage, recevant en entrée la fréquence de référence et délivrant en sortie un signal d'inhibition correspondant à la fin de toute administration du principe actif, le circuit de commande interdisant toute application d'une tension d'électrolyse après apparition de ce signal d'inhibition.

Dans le dispositif de l'invention, la deuxième chambre peut être modifiée de manière à éjecter plusieurs principes actifs et l'ensemble des deux chambres dédoublé avec un système de commande unique,- permettant ainsi l'injection alternative ou simultanée de deux principes actifs ou groupes de principes actifs.

On va maintenant donner un exemple de réalisation de l'invention en référence aux figures annexées.

La figure 1 montre, en coupe, une forme de réalisation de la pompe électrolytique de l'invention.

La figure 2 est une variante de réalisation de la pompe de la figure 1.

La figure 3 montre, sous forme de schéma par blocs, le circuit de commande de la pompe des figures 1 et 2.

La figure 4 est un détail de l'un des sous-ensembles de ce circuit.

La figure 5 est une série de chronogrammes correspondant à différents signaux produits par les circuits des figures 3 et 4.

La figure 6 est un graphique montrant la caractéristique, relevée avec une cellule du type illustré figure 1, du débit volumique en fonction de l'intensité dans la cellule d'électrolyse.

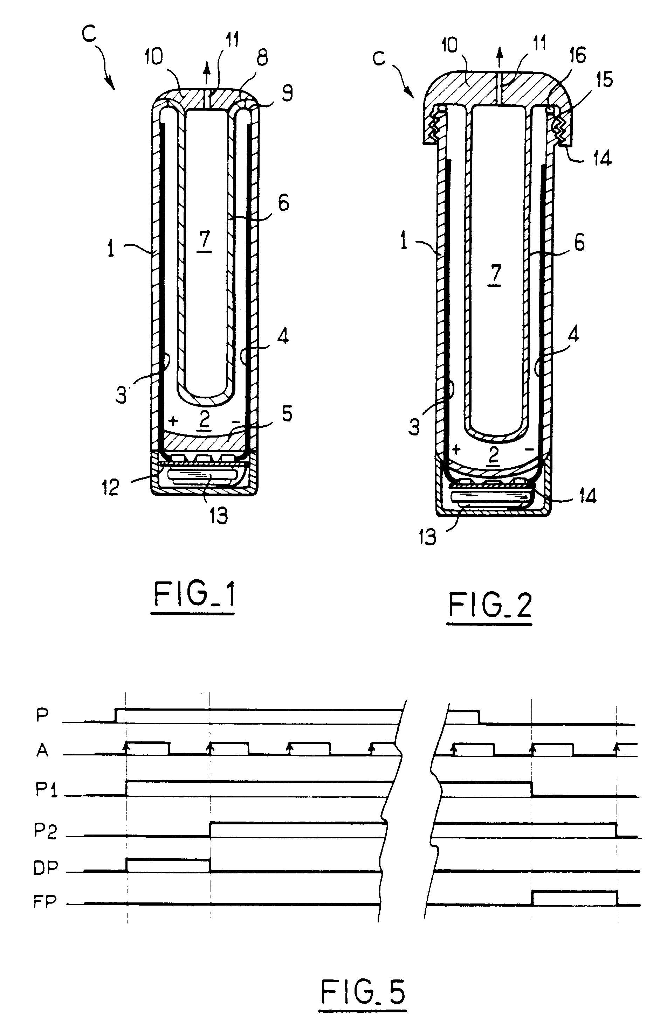

La figure 1 montre la structure générale de la pompe à effet électrolytique de l'invention.

Celle-ci comporte essentiellement une enveloppe tubulaire externe 1, rigide, définissant une première chambre 2 contenant un électrolyte liquide (on notera qu'il est important que l'électrolyte soit liquide si l'on veut pouvoir éjecter des doses ponctuelles avec un très faible temps de réponse). Cette enveloppe extérieure 1 est réalisée en un matériau biocompatible (par exemple une résine silicone) si l'on souhaite que le dispositif puisse être implanté ou ingéré, ce qui sera le plus souvent le cas.

Des électrodes 3, 4 sont placées contre la paroi de l'enveloppe externe 1 et sont avantageusement en forme de fils allongés, par exemple en cuivre (ou tout autre métal adapté, compte tenu de la nature de l'électrolyte), s'étendant sur la quasi-totalité de la longueur du tube 1. En outre, on prévoit plusieurs paires d'électrodes (une seule paire d'électrodes 3, 4, étant visible sur la coupe de la figure 1), toutes disposées parallèlement contre la paroi du tube 1 et réparties régulièrement sur la circonférence de celui-ci avec alternance des polarités d'une électrode à la suivante.

Cette configuration particulière permet d'être sûr que l'on aura toujours, au moins partiellement, une paire d'électrodes qui baignera dans le liquide quelle que soit l'importance du dégagement gazeux et quelle que soit l'orientation dans l'espace du dispositif (ce qui est essentiel dans le cas d'un dispositif implanté ou ingéré).

On peut ainsi prévoir deux, quatre, six ou huit électrodes (ou même plus), ce qui permet d'être sûr que l'on aura un contact électrodes/électrolyte permanent, et que ce contact se fera sur une surface d'électrodes suffisante.

On voit également que cette configuration particulière d'électrode ne crée aucun volume mort à l'intérieur de la cellule d'électrolyse, ce qui permet d'avoir un rapport volume de principe actif/volume du dispositif qui est très élevé, ce qui permet de réduire au minimum les dimensions globales du dispositif (ceci constituant une autre caractéristique essentielle lorsque l'on veut réaliser un dispositif destiné à être implanté ou ingéré).

Le tube 1 contenant l'électrolyte est fermé à l'une de ses extrémités (l'extrémité inférieure 5, avec l'orientation de la figure 1) et reçoit en son centre, sur la majeure partie de sa longueur, une poche souple 6 définissant une seconde chambre 7 renfermant le principe actif.

La poche 6 est en forme de doigt de gant, et elle est réalisée en un matériau déformable (par exemple un élastomère) chimiquement neutre vis-à-vis à la fois de l'électrolyte et du principe actif et imperméable au gaz (hydrogène) dégagé par le phénomène d'électrolyse.

La seconde chambre 7 présente, du côté de son ouverture, un rebord 8 qui est relié de façon étanche (par exemple par collage) au rebord homologue 9 du tube rigide extérieur 1, de manière à clore complètement le volume de la première chambre 2.

Quant à la seconde chambre 7, celle-ci est obturée par un élément 10 pourvu d'un capillaire 11 destiné à permettre l'éjection du principe actif (ce capillaire 11 pouvait être, en variante, remplacé par un tube permettant la délivrance du produit en un point distant de la pompe).

Enfin, le dispositif comprend un circuit électrique de commande 12, que l'on décrira plus bas, alimenté par une pile 13 servant de source d'énergie pour l'électrolyse. Avantageusement, les organes électriques 12, 13 peuvent être disposés à l'une des extrémités du tube, comme illustré sur la figure.

On notera également que, grâce à la très grande surface de la paroi souple 6, on obtient une compression uniforme, isostatique, du principe actif, avec une très faible résistance mécanique à la transmission de la pression du gaz produit dans la première chambre 2 vers le principe actif contenu dans la seconde chambre 7. Ainsi, les pics de pression produits par un contrôle approprié de l'électrolyse seront fidèlement transmis au volume de principe actif et se traduiront en pics de débit de principe actif expulsé par le capillaire 11.

La figure 2 montre une variante de réalisation dans laquelle la poche souple 6 et l'obturateur 10 sont formés d'une seule pièce par un élément en forme de bouchon à vis dont le taraudage 14 vient s'adapter sur un filetage 15 du tube extérieur 1, l'étanchéité étant assurée par un joint torique 16. Les autres éléments sont semblables à ceux de la figure 1, et ont été désignés par les mêmes références numériques.

Cette configuration modifiée rend la cellule aisément réutilisable par simple dévissage de l'élément 10, ce qui permet d'échanger la solution d'électrolyse usagée par une solution neuve, la poche 6 étant rechargée d'une nouvelle dose de principe actif par injection au travers du capillaire 11.

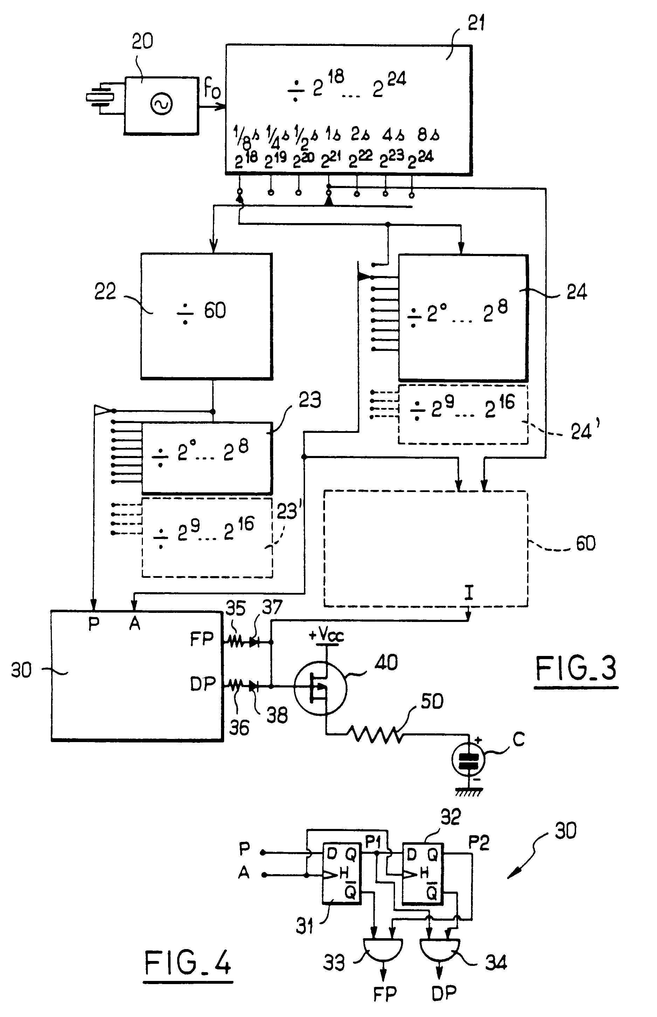

La figure 3 montre le circuit de commande permettant de réaliser des éjections séquentielles de brèves doses contrôlées de principe actif.

Ce circuit comporte essentiellement un oscillateur à quartz 20 délivrant une fréquence de référence fₒ, par exemple de 2,097 MHz. Cette fréquence de référence est appliquée à un circuit diviseur 21 dont le rang de division peut être sélectionné entre 2¹⁸ et 2²⁴, donnant ainsi des périodes comprises entre 1/8^{e} de seconde et 8 s.

Un premier ensemble de circuits 22, 23, 23' permet de générer un signal P (désigné "signal de période") définissant la périodicité des éjections de produit, c'est-à-dire l'intervalle de temps séparant deux éjections successives. Ces circuits comportent un diviseur par soixante référencé 22, avec en cascade un diviseur à rang programmable compris entre 1 et 2⁸. Si l'on applique en entrée du diviseur 22 des signaux de période 1 s, on peut ainsi obtenir en sortie du diviseur 23 des signaux P de périodes comprises entre 7,5 s et 3448 mn (57 h 28 mn).

Si on le souhaite, on peut augmenter encore ces durées en prévoyant un second circuit 23', identique au circuit 23, permettant d'obtenir des divisions comprises entre 2⁹ et 2¹⁶.

Outre le signal P définissant la périodicité des éjections, il est également nécessaire de produire un signal A (désigné "signal d'activation") définissant la durée propre de chacune des éjections); ce signal d'activation A est obtenu à partir d'un diviseur 24, de rang compris entre 1 et 2⁸, recevant les impulsions de périodes 1/8^{e} de seconde en sortie du diviseur 21 et délivrant donc un signal A formé d'impulsion de durées comprises entre 1/8^{e} de seconde et 2048 s (34 mn 8 s); comme dans le cas du signal de période, la durée du signal d'activation peut être prolongée en prévoyant un étage supplémentaire de division 24', identique à l'étage 24 et en cascade avec celui-ci.

Les deux signaux de période P et d'activation A sont appliqués conjointement à un circuit 30 qui les combine pour donner deux signaux "fin de période" FT et "début de période" DP qui commanderont tour à tour l'activation de la cellule d'électrolyse; pour respecter la périodicité des éjections que l'on s'est donnée, il est en effet nécessaire de commander la cellule électrolytique non pas sur l'état du signal de période P mais sur les changements d'états de celui-ci : le signal DP correspond ainsi à un front montant et le signal FP à un front descendant, et chacun de ces signaux devra commander l'activation de la cellule électrolytique.

La figure 4 montre le détail du circuit de commande 30 : celui-ci comprend deux flip-flops de type D 31 et 32 recevant comme signal d'horloge le signal d'activation A. Le signal de période P est appliqué à l'entrée D du premier flip-flop 31, qui donne sur sa sortie Q un signal P₁, appliqué à l'entrée D du second flip-flop 32, qui délivre sur sa sortie Q un signal P₂. Les signaux de sortie Q et Q̅ respectifs de chacun des deux flip-flops 31 et 32 sont combinés de la manière illustrée par deux portes ET 33 et 34 pour donner les signaux FP et DP qui commanderont l'activation de la cellule d'électrolyse.

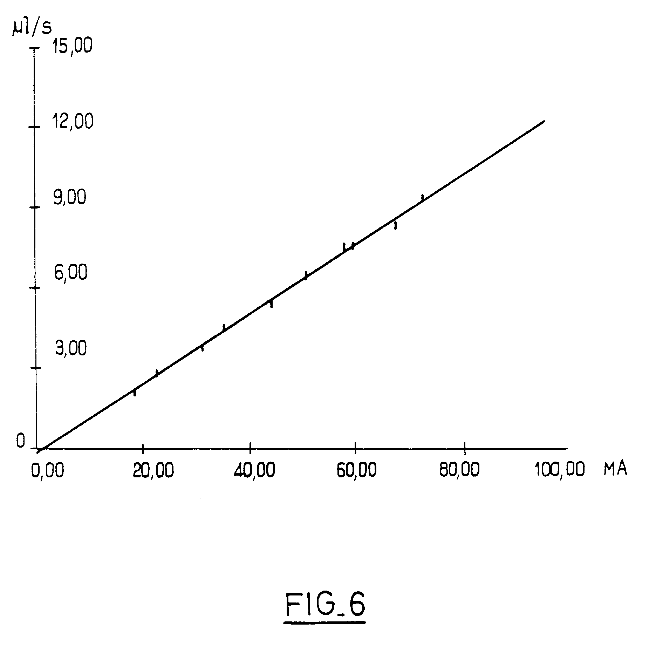

Le chronogramme de la figure 5 illustre la succession des différents signaux produit par ce circuit de commande 30.

Les signaux FP et DP sont appliqués, via des résistances 35, 36 et des diodes 37, 38 (qui, ensemble, constituent l'équivalent d'une porte OU), à la base d'un transistor de puissance 40 dont la source est reliée à la tension d'alimentation +V_{cc} et dont le drain est relié à l'une des électrodes (ou l'une des séries d'électrodes de la paire d'électrodes) de la cellule électrolytique C par l'intermédiaire d'une résistance 50.

La résistance 50 a une valeur notablement supérieure à la résistance de l'électrolyte, par exemple une valeur de l'ordre de 200Ω , ce qui permet de neutraliser l'effet de la résistance propre de la cellule électrolytique qui évolue au cours du temps en raison de la modification de la composition de l'électrolyte et de la polarisation des électrodes.

Ceci permet de stabiliser le courant d'électrolyse malgré la variation des caractéristiques physicochimiques propres de la cellule électrolytique et de conserver, lorsque la cellule d'électrolyse est alimentée, une intensité d'électrolyse constante et donc un débit constant d'éjection du principe actif même après une très longue durée de fonctionnement.

De préférence, l'électronique de commande comporte également un circuit d'inhibition 60 permettant, au bout d'un certain temps prédéterminé, d'interdire toute activation de la cellule d'électrolyse et donc de mettre fin définitivement à la délivrance du principe actif.

Le circuit 60 peut être par exemple un circuit diviseur fonctionnant en décompteur, recevant en entrée soit des impulsions délivrées en sortie du circuit 21 (lorsque l'on souhaite arrêter la pompe après une durée de fonctionnement fixe, prédéterminée), soit à partir du signal d'activation (lorsque l'on souhaite arrêter la pompe après la délivrance d'un nombre prédéterminé de doses ponctuelles). Une fois atteint le seuil que l'on s'est fixé, le circuit 60 peut par exemple délivrer un signal d'inhibition I au niveau bas, ce qui correspond à une mise en permanence au potentiel de la masse de la base du transistor 40, inhibant ainsi toute activation ultérieure de la cellule d'électrolyse (les signaux FP et DP devenant alors sans effet).

En variante, il est également possible de réaliser l'inhibition en court-circuitant la pile, ce qui est une solution meilleure du point de vue de la sécurité mais ne permet pas de récupérer la pile si l'on souhaite récupérer et réutiliser le dispositif.

On peut également choisir une pile telle que l'énergie totale qu'elle peut délivrer soit légèrement inférieure à l'énergie totale nécessaire pour évacuer la totalité du principe actif contenu dans la chambre déformable 7.

On évite ainsi que l'électrolyse ne se prolonge même après expulsion complète du produit, ce qui pourrait présenter des risques importants du fait d'un dégagement excessif d'hydrogène et, corrélativement, d'une élévation excessive de pression à l'intérieur du dispositif.

Pour réduire au minimum la consommation propre de l'électronique de commande, on choisit pour celle-ci des circuits CMOS et un transistor MOSFET de puissance, pouvant être commandé directement par la logique CMOS.

Bien évidemment, la réalisation de ces circuits ne se limite pas, comme illustré, à une logique câblée à éléments discrets; ils pourraient tout aussi bien être réalisés sous forme d'une puce unique en logique câblée, ou même en logique microprogrammée. Dans ce dernier cas, on pourrait en particulier utiliser des programmes (en ROM, EPROM ou EEPROM) pour définir le séquencement choisi et pour permettre également de modifier certaines valeurs des paramètres essentiels et les adapter en fonction des besoins.

On notera également que le système pourrait, de manière en elle-même connue, être initialisé et déclenché par télécommande depuis un équipement extérieur.

La figure 6 montre un relevé expérimental de la caractéristique donnant le débit de principe actif (en »l/s) en fonction du courant d'électrolyse (en mA). Le principe actif était de la gentamycine en solution dans l'eau à 2 Kg/l.

On peut constater l'excellente linéarité obtenue, et donc la possibilité d'ajuster précisément le débit d'éjection du principe actif par contrôle du courant d'électrolyse (c'est-à-dire par ajustement de la résistance série 50).

Outre la très bonne corrélation débit/intensité dans tous les cas de figure, l'expérimentation a montré également que l'on obtient, pour une intensité donnée, des valeurs de débit très proches pour des principes actifs de viscosités très différentes, ce qui permet d'envisager l'utilisation avec des principes actifs de natures très différentes.

## Revendications

1. Dispositif de délivrance d'un principe actif pharmacologique par pompage électrolytique, ce dispositif comprenant une cellule d'électrolyse (C) comportant :
- une première chambre (2) à volume variable, close, affectant une forme tubulaire (1), renfermant un électrolyte liquide baignant des électrodes (3, 4) de forme allongée et produisant un dégagement gazeux sous l'effet d'une tension d'électrolyse appliquée au niveau des électrodes (3, 4) en contact avec cet électrolyte, ce dégagement gazeux provoquant une expansion de la première chambre, et
- une seconde chambre (7) à volume variable renfermant le principe actif, cette chambre débouchant vers l'extérieur par un orifice (11) permettant la délivrance du principe actif, le volume total des deux chambres (2, 7) étant constant, de sorte que l'expansion de la première chambre provoque une diminution de volume de la seconde chambre et une expulsion corrélative, contrôlée, du principe actif que renferme cette dernière,
- les deux chambres (2, 7) étant séparées l'une de l'autre par une paroi souple (6) subissant la pression résultant du dégagement gazeux de manière à réduire le volume de la seconde chambre (7) et en expulser le principe actif,
dispositif caractérise en ce que :
- les electrodes (3, 4) sont disposées contre la paroi intérieure du tube (1) et s'étendent sur la majeure partie de la longueur de cette première chambre de manière à se trouver toujours au moins partiellement en contact avec l'électrolyte quelle que soit la position du dispositif, et
- la seconde chambre (7) à volume variable s'étend axialement sur la majeure partie de la longueur de ladite première chambre (2).

2. Le dispositif de la revendication 1, dans lequel la première chambre (2) est définie par un tube rigide (1) fermé à l'une de ses extrémités (5), ce tube enfermant une poche souple centrale s'étendant axialement sur la majeure partie de la longueur du tube et définissant la seconde chambre (7), le rebord (8) de cette poche étant relié au tube rigide à l'extrémité (9) opposée à son extrémité fermée et étant fermé par un obturateur (10) comportant l'orifice (11) de délivrance du principe actif.

3. Le dispositif de la revendication 2, dans lequel les électrodes (3, 4) sont disposées contre la paroi intérieure du tube (1) et s'étendent sur la majeure partie de la longueur de celui-ci.

4. Le dispositif de la revendication 3, comprenant une pluralité de paires d'électrodes (3, 4) semblables, réparties circonférentiellement de façon régulière contre la paroi intérieure du tube (1) avec alternance des polarités d'une électrode à la suivante.

5. Le dispositif de la revendication 1, comprenant, en série dans le circuit de la cellule d'électrolyse (C), une résistance (50) de valeur notablement supérieure à la résistance propre, prise entre les électrodes, de l'électrolyte.

6. Le dispositif de la revendication 1, dans lequel la tension d'électrolyse est appliquée aux électrodes de façon intermittente, de manière à délivrer le principe actif par doses prédéterminées répétées, administrées à intervalles réguliers.

7. Le dispositif de la revendication 6, comprenant un circuit de contrôle d'électrolyse comprenant :
- une source de tension d'électrolyse (+V_{cc}),
- un oscillateur (20), délivrant une fréquence de référence prédéterminée,
- des premiers moyens de comptage (21, 22, 23), recevant en entrée la fréquence de référence (fₒ) et délivrant en sortie un signal périodique de séquencement (P) définissant la périodicité d'administration de la dose de principe actif,
- des seconds moyens de comptage (21, 24), recevant en entrée la fréquence de référence (fₒ) et délivrant en sortie un signal périodique d'activation (A) définissant la durée d'administration de chaque dose de principe actif, et
- un circuit de commande (30-40), combinant ces signaux de séquencement et d'activation de manière a commander sélectivement l'application de la tension d'électrolyse (+V_{cc}) aux bornes de al cellule d'électrolyse (C) de manière a réaliser la délivrance du principe actif avec la périodicité et la durée voulues.

8. Le dispositif de la revendication 7 , dans lequel le circuit de contrôle d'électrolyse comprend en outre des troisièmes moyens de comptage (21, 60), recevant en entrée la fréquence de référence (fₒ) et délivrant en sortie un signal d'inhibition (I) correspondant à la fin de toute administration du principe actif, le circuit de commande (30-40) interdisant toute application d'une tension d'électrolyse après apparition de ce signal d'inhibition.

9. Le dispositif selon l'une des1 revendications 1 à 8, caractérisé en ce que la seconde chambre (7) est agencée sous la forme d'une chambre à deux compartiments dont chacun est muni de son propre orifice (11) de délivrance de principe actif.

10. Le dispositif selon la revendication 9 , caractérisé en ce qu'il comporte un circuit de contrôle d'électrolyse commun à deux dispositifs de délivrance comprenant chacun une première et une seconde chambres (2, 7), en vue de permettre la délivranc alternative de principes actifs différents.

## Claims

1. A device for delivering a pharmacologically active principle by electrolytic pumping, the device including an electrolysis cell (C) comprising:
a closed variable volume first chamber (2) which is tubular in shape (1) containing a liquid electrolyte in which electrodes (3, 4) of elongate shape are immersed and giving off gas under the effect of an electrolysis voltage applied to electrodes (3, 4) in contact with the electrolyte, the gas given off causing the first chamber to expand; and
a variable volume second chamber (7) containing the active principle, said second chamber opening out to the outside via an orifice (11) enabling the active principle to be delivered, the total volume of the two chambers (2, 7) being constant, such that expansion of the first chamber causes the volume of the second chamber to be reduced and gives rise to a corresponding controlled expulsion of the active principle contained therein; and
the two chambers (2, 7) being separated from each other by a flexible wall (6) subjected to the pressure resulting from gas being given off in such a manner as to reduce the volume of the second chamber (7) and expel the active principle;
the device being characterized in that:
the electrodes (3, 4) are disposed against the inside wall of the tube (1) and extend over the major portion of the length of said first chamber so that the electrodes are always at least in part in contact with the electrolyte regardless of the position of the device; and
the variable volume second chamber (7) extends axially over the major portion of the length of said first chamber (2).

2. The device of claim 1, in which the first chamber (2) is defined by a rigid tube (1) closed at one of its ends (5), the tube containing a central flexible bag extending axially over the major portion of the length of the tube and defining the second chamber (7), the rim (8) of the bag being connected to the rigid tube at its end (9) opposite to its closed end and being closed by a plug (10) including the orifice (11) through which the active principle is delivered.

3. The device of claim 2, in which the electrodes (3, 4) are disposed against the inside wall of the tube (1) and extend over the major portion of the length thereof.

4. The device of claim 3, including a plurality of similar pairs of electrodes (3, 4) distributed circumferentially in regular manner against the inside wall of the tube (1) with electrode polarity alternating from one electrode to the next.

5. The device of claim 1, including a resistor (50) in series in the electrolysis cell circuit (C), the resistance of the resistor being significantly higher than the resistance of the electrolyte as measured between the electrodes.

6. The device of claim 1, in which the electrolysis voltage is applied to the electrodes intermittently so as to deliver the active principle in repeated predetermined doses which are administered at regular intervals.

7. The device of claim 6, including an electrolysis control circuit comprising:
a source of electrolysis voltage (+Vcc);
an oscillator (20) delivering a predetermined reference frequency;
first counter means (21, 22, 23) receiving the reference frequency (fo) at an input and delivering a periodic sequencing signal (P) at an output defining the periodicity with which a dose of the active principle is administered;
second counter means (21, 24) receiving the reference frequency (fo) at an input and delivering a periodic activation signal (A) at an output defining the length of time during which each dose of active principle is administered; and
a control circuit (30-40) combining the sequencing signal and the activation signal in such a manner as to selectively control application of the electrolysis voltage (+Vcc) to the terminals of the electrolysis cell (C) so as to cause the active principles to be delivered with the desired periodicity and for the desired length of time.

8. The device of claim 7, in which the electrolysis control circuit further includes third counter means (21, 60) receiving the reference frequency (fo) at an input and delivering an inhibit signal (I) at an output, the inhibit signal corresponding to the end of administering any active principle, the control circuit (30-40) preventing any application of an electrolysis voltage after the inhibit signal has appeared.

9. The device according to any one of claims 1 to 8, characterized in that the second chamber (7) is in the form of a chamber having two compartments each of which is provided with its own orifice (11) for delivering active principles.

10. The device according to claim 9, characterized in that it includes an electrolysis control circuit common to two delivery devices each comprising a first chamber and a second chamber (2, 7) for the purpose of enabling different active principles to be delivered alternately.

## Patentansprüche

1. Vorrichtung zur Abgabe (Austeilung) eines pharmakologischen Wirkstoffes durch elektrolytisches Pumpen, die eine Elektrolysezelle (C) umfaßt, die aufweist
- eine geschlossene erste Kammer (2) mit variablem Volumen, die eine rohrförmige Gestalt hat und eine Elektrolytflüssigkeit enthält, in die Elektroden (3, 4) einer länglichen Form eintauchen, und in der unter der Einwirkung einer an die Elektroden (3, 4) angelegten Elektrolysespannung im Kontakt mit diesem Elektrolyten ein Gas freigesetzt wird, das eine Expansion der ersten Kammer hervorruft, und
- eine zweite Kammer (7) mit einem variablen Volumen, die den Wirkstoff enthält, wobei diese Kammer sich durch eine Öffnung (11) nach außen öffnet, welche die Abgabe des Wirkstoffes erlaubt, wobei das Gesamtvolumen der beiden Kammern (2, 7) konstant ist, so daß die Expansion der ersten Kammer eine Verkleinerung des Volumens der zweiten Kammer und eine korrelative, kontrollierte Ausstoßung des Wirkstoffes, der in der letzteren enthalten ist, bewirkt wird,
- wobei die beiden Kammern (2, 7) durch eine weiche Wand (6) voneinander getrennt sind, die dem aus der Gasfreisetzung resultierenden Druck ausgesetzt ist, so daß das Volumen der zweiten Kammer (7) abnimmt und der Wirkstoff daraus ausgestoßen wird, dadurch gekennzeichnet, daß
- die Elektroden (3, 4), die an der Innenwand des Rohres (1) angeordnet sind, sich über den größten Teil der Länge dieser ersten Kammer so erstrecken, daß sie immer mindestens teilweise im Kontakt stehen mit dem Elektrolyten, unabhängig von der Position der Vorrichtung, und
- die zweite Kammer (7) mit variablem Volumen sich in axialer Richtung über den größten Teil der Länge der genannten ersten Kammer (2) ersteckt.

2. Vorrichtung nach Anspruch 1, in der die erste Kammer (2) durch ein an einem seiner Enden (5) verschlossenes starres Rohr (1) definiert ist, das eine zentrale weiche Tasche umschließt, die sich in axialer Richtung über den größten Teil der Länge des Rohres erstreckt, und die zweite Kammer (7) definiert, wobei der umgebogene Rand (8) dieser Tasche mit dem starren Rohr verbunden ist am Ende (9), das einem geschlossenen Ende gegenüberliegt, und durch einen Verschluß (10) verschlossen ist, der die Öffnung (11) für die Abgabe des Wirkstoffes aufweist.

3. Vorrichtung nach Anspruch 2, in der die Elektroden (3, 4) an der Innenwand des Rohres (1) angeordnet sind, und sich über den größten Teil der Länge desselben erstrecken.

4. Vorrichtung nach Anspruch 3, die eine Vielzahl von ähnlichen Elektrodenpaaren (3, 4) umfaßt, die an der Innenwand des Rohres (1) gleichmäßig um den Umfang herum verteilt sind mit wechselnden Polaritäten von einer Elektrode zur nächsten.

5. Vorrichtung nach Anspruch 1, die in Reihe in dem Stromkreis der Elektrolysezelle (C) einen Widerstand (50) mit einem deutlich höheren Wert als der Eigenwiderstand, gemessen zwischen den Elektroden des Elektroyten, aufweist.

6. Vorrichtung nach Anspruch 1, in der die Elektrolysespannung intermittierend an die Elektroden angelegt wird, so daß der Wirkstoff in wiederkehrenden vorgegebenen Dosen, die in regelmäßigen Zeitabständen verabreicht werden, abgegeben wird.

7. Vorrichtung nach Anspruch 6, die einen Elektrolyse-Regel-Stromkreis aufweist mit
- einer Elektrolyse-Spannungsquelle (+V_{cc}),
- einem Oszillator (20), der eine vorgegebene Bezugsfrequenz abgibt,
- ersten Zähleinrichtungen (21, 22, 23), die am Eingang die Bezugsfrequenz (fₒ) aufnehmen und am Ausgang ein periodisches Sequenzierungs-Signal (P) abgeben, das die Verabreichungsperiodizität der Dosis an Wirkstoff definiert,
- zweiten Zähleinrichtungen (21, 24), die am Eingang die Bezugsfrequenz (fₒ) aufnehmen und am Ausgang ein periodisches Aktivierungssignal (A) abgeben, das die Dauer der Verabreichung jeder Dosis des Wirkstoffes definiert, und
- einem Steuerstromkreis (30-40), der diese Sequenzierungs-Signale und Aktivierungssignale so miteinander kombiniert, daß das Anlegen der Elektrolysespannung (+V_{cc}) an die Anschlußklemmen der Elektrolysezelle (C) selektiv gesteuert wird, so daß die Abgabe des Wirkstoffes mit der gewünschten Periodizität und der gewünschten Dauer erfolgt.

8. Vorrichtung nach Anspruch 7, in der der Elektrolyse-Regel-Stromkreis außer dritten Zähleinrichtungen (21, 60), die am Eingang die Bezugsfrequenz (fₒ) aufnehmen und am Ausgang ein Inhibierungssignal (I) abgeben, das dem Ende jeder Verabreichung des Wirkstoffes entspricht, einen Steuer-Stromkreis (30-40) aufweist, der jedes Anlegen einer Elektrolysespannung nach dem Auftreten dieses Inhibierungssignals verbietet.

9. Vorrichtung nach einem der Ansprüche 1 bis 8, dadurch gekennzeichnet, daß die zweite Kammer (7) die Form einer Kammer mit zwei Abteilen hat, von denen jedes mit einer eigenen Öffnung (11) für die Abgabe des Wirkstoffes ausgestattet ist.

10. Vorrichtung nach Anspruch 9, dadurch gekennzeichnet, daß sie einen Elektrolyse-Regel-Stromkreis aufweist, der beiden Abgabe-Vorrichtungen gemeinsam ist, die jeweils eine erste Kammer und eine zweite Kammer (2, 7) umfassen, um die alternierende Abgabe verschiedener Wirkstoffe zu ermöglichen.
